# FASCICULE DE BREVET EUROPEEN

(11) **EP 2 682 078 B1**
(45) Date de publication et mention de la délivrance du brevet: **09.09.2015**
(21) Numéro de dépôt: 13290131.5
(22) Date de dépôt: 10.06.2013
(51) Int. Cl.: A61F 9/007

(54) **Canule à fond biseauté**
Kanüle mit abgeschrägter Öffnung
Cannula with a slanted opening

(30) Priorité: 06.07.2012 FR 1201919
(43) Date de publication de la demande: 08.01.2014
(73) Titulaire: France Chirurgie Instrumentation SAS-FCI, 75015 Paris (FR)
(72) Inventeur: Urion, Stéphane, 70190 Cromary (FR)
(74) Mandataire: Eidelsberg, Olivier Nathan

(56) Documents cités:
- EP-A1- 0 623 329
- US-A- 6 117 116

## Description

La présente invention se rapporte à une canule d'intubation pour un système d'intubation, notamment monocanaliculaire ou bicanaliculaire, dit de Ritleng, qui comporte une canule d'intubation, un fil de guidage et une sonde, notamment en forme de tube, en particulier en silicone, cette dernière étant destinée à être insérée dans le canal lacrymal. La présente invention se rapporte aussi à un système d'intubation de ce genre.

L'objectif est d'introduire la sonde en forme de tube en silicone dans les voies lacrymales. Pour se faire, on procède d'abord au cathétérisme des voies lacrymales du patient à l'aide de la canule ouverte à une extrémité, fermée à l'extrémité opposée et comportant une fente s'étendant le long d'une génératrice de l'extrémité ouverte vers l'extrémité fermé, jusqu'à une lumière formée dans la paroi latérale de la canule, lumière qui a une plus grande dimension en largeur (mesurée perpendiculairement à l'axe longitudinal de la canule) que la fente. Ensuite, le fil relié au tube en silicone est introduit à l'intérieur de la canule et est poussé hors de celle ci à travers la dite lumière jusque dans la fosse nasale. Le fil, classiquement en prolène, est alors récupéré dans la fosse nasale et on retire la canule des voies lacrymales en la faisant coulisser sur le fil et en la désolidarisant au niveau d'un tronçon aminci de ce dernier en faisant sortir ce tronçon aminci par la fente formée sur la longueur de la canule. Une fois la canule retirée, on tire sur le fil en prolène par le côté de la fosse nasale pour faire entrer la sonde tube en silicone, solidarisée à l'autre extrémité du fil, dans les voies lacrymales. Un système de Ritleng de ce genre est décrit dans le brevet européen EP 0623329. L'extrémité du fil, de diamètre 0,4 mm, y est facilement insérée dans la canule, de diamètre 0,5 mm et est facilement poussée jusqu'à venir en contact avec le fond de la canule, à l'opposée de l'ouverture par laquelle est introduit le fil, au delà de la lumière.

Bien que ce système de Ritleng ait présenté une avancée certaine pour l'introduction de la sonde tube de silicone dans les voies lacrymales, on souhaite améliorer encore plus cette introduction, notamment simplifier la tâche du chirurgien et la rendre moins traumatique pour le patient.

La présente invention vise à surmonter les inconvénients de l'art antérieur en proposant une canule d'un système de Ritleng suivant la revendication 1, des perfectionnements étant définis aux sous revendications 2 à 4.

Ainsi, le chirurgien, lorsqu'il fait coulisser le fil de guidage dans la canule pour en faire ressortir l'extrémité libre par le côté de la fosse nasale par la lumière, n'a pas de difficulté pour accéder à cette extrémité libre qui, contrairement au cas des canules de l'art antérieur (voir notamment EP 062329), ne peut pas être piégée dans l'espace intérieur de la canule au delà de la lumière à l'opposée de l'ouverture d'introduction du fil, entre la lumière et le fond de la canule, le fond intérieur de la canule se trouvant au niveau de la fin de la lumière et formant une rampe de guidage pour le fil bers l'extérieur. L'opération de récupération par le praticien de l'extrémité du fil est ainsi moins traumatique pour le patient, le chirurgien n'ayant pas à trifouiller dans la fosse nasale pour aller chercher le fil piégé dans la canule. L'opération est donc également plus simple pour le chirurgien.

L'espace entre la fin de la lumière dans la direction longitudinale vers l'extrémité extérieure fermée et l'extrémité extérieure fermée est plein de sorte que la paroi intérieure de fond de la canule se trouve sensiblement au niveau de la fin de la lumière.

La canule comporte une paroi intérieure formant fond de la canule qui ferme l'espace entre la fin de la lumière dans la direction de l'extrémité extérieure fermée et cette extrémité extérieure fermée.

La paroi intérieure de fond de la canule qui se trouve sensiblement au niveau de la fin de la lumière dans la direction longitudinale vers l'extrémité fermée extérieure fait un angle inférieur à 90°, notamment compris entre 15 et 30° avec l'axe longitudinal, pour ainsi former une rampe de guidage vers le haut, c'est à dire vers la lumière, pour l'extrémité du fil que l'on fait coulisser dans la canule en la poussant par l'ouverture de la canule.

La présente invention se rapporte également à un système dit de Ritleng comportant une canule suivant l'invention et une sonde d'intubation comportant au moins une partie en forme de tube en un matériau souple biocompatible, notamment en silicone, solidarisée à un fil de guidage, notamment en prolène, dont une extrémité, pour solidariser mutuellement la sonde et le fil, est introduite dans le tube par une ouverture de ce dernier.

De préférence le tube de la sonde comporte un tronçon, du côté de l'ouverture du tube par laquelle l'extrémité du fil est introduite dans le tube, ayant une forme en biseau, le diamètre extérieur du tube, le long du tronçon, augmentant depuis l'ouverture.

De préférence, l'épaisseur du tube augmente depuis l'ouverture du tube le long du tronçon en biseau puis est constante après cela.

En prévoyant ainsi une forme en biseau de la partie en forme de tube de la sonde qui vient à la suite du fil solidarisé à la sonde, on s'assure qu'au passage entre le fil et le tube en silicone la transition se fait plus en douceur et par conséquent lorsqu'on tire la sonde par le fil pour la faire passer dans les voies lacrymales, le tube, grâce à cette forme en biseau, pénètre plus facilement en suivant plus facilement le fil guide, notamment sans que le bord du tube ne se retourne sur lui-même, et ce en étant moins traumatique pour les parois du canal lacrymal.

A titre d'exemple, on décrit maintenant un mode de réalisation de l'invention en se rapportant aux dessins dans lesquels :
- la Figure 1: est une vue de dessus d'une canule d'un système d'intubation suivant l'invention ;
- la Figure 2: représente un ensemble d'intubation formant sonde de Ritleng suivant l'invention, dans lequel on voit bien la liaison entre le tube de silicone et le fil de guidage ;
- la Figure 3: est une vue schématique décrivant le procédé d'introduction du tube de silicone dans le canal lacrymal ;
- la Figure 4: représente un autre mode de réalisation d'une canule d'un système d'intubation suivant l'invention, vu en coupe longitudinale ; et
- la Figure 5: est une vue de dessus de la canule de la figure 4.

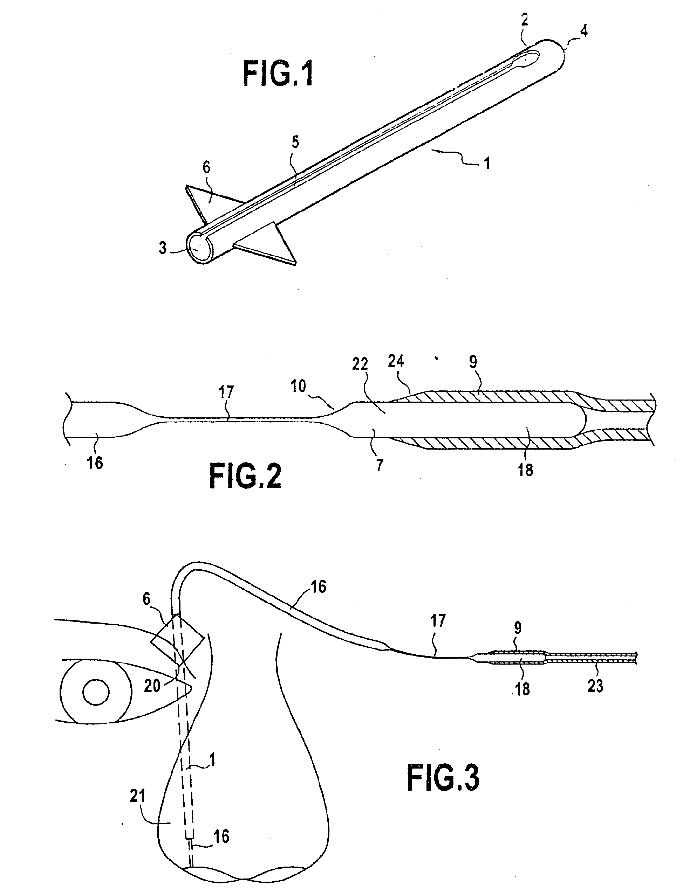

A la Figure 1, il est représenté une canule d'un système suivant l'invention. Cette canule 1 est constituée d'un corps cylindrique circulaire creux en matière semi rigide, par exemple en matière thermoplastique, notamment en PEEK ou en polyarylamide, ouvert à une extrémité 3 proximale et fermée à l'extrémité 4 opposée distale.

La canule 1 comporte une fente 5 longitudinale qui s'étend le long d'une génératrice du cylindre, de l'ouverture 3 proximale ouverte en direction de l'extrémité 4 fermée, jusqu'à une lumière 2 de plus grande largeur (mesurée perpendiculairement à l'axe longitudinal du cylindre, qui est parallèle à la fente longitudinale) que celle de la fente 5.

La lumière 2 a, vue de dessus comme à la figure 1, une forme en ellipse dont le petit diamètre est perpendiculaire à l'axe longitudinal et le grand diamètre parallèle à l'axe de la fente. Le petit diamètre est plus grand que l'épaisseur de l'interstice de la fente.

Des ailettes 6 de préhension font saillie latéralement de la canule, au voisinage de l'ouverture 3 proximale.

A la Figure 2, il est représenté en partie un ensemble 10 d'intubation dit de Ritleng. Cet ensemble 10 d'intubation dit de Ritleng est constitué d'un fil 7, notamment en prolène ou en un autre matériau biocompatible et souple analogue, et d'une sonde tube 9 en silicone.

Le fil comporte trois tronçons, à savoir un tronçon 16 d'extrémité de grand diamètre suivi d'un tronçon 17 intermédiaire de plus petit diamètre, lui-même suivi d'un tronçon 18 d'extrémité opposé de grand diamètre. Le diamètre du tronçon 16 de grand diamètre est tel qu'il peut passer dans le tube et dans la lumière 2 mais pas dans la fente 5. Le diamètre du tronçon 17 intermédiaire de plus petit diamètre est tel qu'il peut passer dans la fente 5.

La sonde 9 est constituée d'un tube formé par un cylindre circulaire creux dont la paroi a une épaisseur comprise de préférence entre 0,15 mm. et 0,5 mm Elle est notamment réalisée en silicone ou autre matériau analogue.

Le tronçon 18 d'extrémité opposé de grand diamètre est inséré dans le tube 9 en silicone pour les solidariser mutuellement. Cette solidarisation peut être effectuée par une insertion à force ou à adaptation serrée. On peut également les solidariser par collage ou tout autre procédé analogue, par exemple soudure à ultrason.

L'insertion de la sonde tube 9 dans le canal lacrymal s'effectue de la manière suivante :

On insère d'abord la canule 1 dans le canal lacrymal en y pénétrant par l'entrée 20 extérieure du côté de l'oeil jusqu'à la sortie 21 débouchant dans la fosse nasale.

Une fois la canule insérée, on y fait coulisser le tronçon 16 jusqu'à ce que l'extrémité du tronçon ressorte de la canule par la lumière 2. Le chirurgien saisit alors cette extrémité ressortie du tronçon 16 et en la tenant par exemple avec une pince adaptée (non représentée) il tire par les ailettes la canule 1 hors du canal lacrymal en Pour insérer le tube 9 sonde dans le canal lacrymal, on insère le tronçon 18 d'extrémité opposé de grand diamètre dans la canule et en prenant cette dernière par les ailettes 4, on insère l'ensemble (canule et fil) dans le canal lacrymal à partir de l'extérieur du côté de l'oeil jusqu'à ce qu'une extrémité du fil passant par la lumière 2 de la canule ressorte par l'intérieur des narines. On retire alors la canule 1 en tirant par les ailettes 4. La canule glisse le long du fil jusqu'à atteindre le tronçon 17 intermédiaire qui peut alors être passé par la fente 5 de la canule pour libérer cette dernière de sa coopération avec le fil. Une fois la canule 1 retirée, on tire le fil par son extrémité ressortant du canal lacrymal du côté de la narine jusqu'à ce que le tube 9 en silicone, solidaire du tronçon 18 du fil, vienne s'insérer dans le canal lacrymal. Une fois le tube 9 en silicone inséré dans le canal lacrymal, on coupe le fil de guidage qui pend à l'extérieur des narines.

La sonde 9 est ici constituée d'un tube ouvert des deux côtés. Du côté de l'ouverture 22 par laquelle le tronçon 16 est introduit dans le tube 9 pour les solidariser mutuellement, la paroi latérale 23 définissant le tube a une épaisseur qui varie de sorte qu'un tronçon (24) de bord du tube 9 ait une forme en biseau.

Lors de la mise en place du tube 9 en silicone par tirage du fil de guidage, le fait que le bord d'attaque du tube 9 de guidage ait une forme en biseau permet d'aider à la mise en place du tube 9 en silicone en faisant en sorte qu'il n'y ait pas, comme dans l'art antérieur, de bord d'attaque en arête qui vienne "taper" contre la paroi du canal lacrymal évitant ainsi soit d'endommager cette paroi du canal lacrymal et/ou soit un retournement du bord du tube 9 pouvant entraîner une désolidarisation du tronçon 16 d'avec le tube 9 avant d'avoir atteint la position finale souhaitée de ce dernier dans le canal lacrymal et donc un mauvais positionnement du tube 9 en silicone nécessitant d'aller le rechercher, puis de recommencer toute l'opération avec un nouveau tube en silicone. Ainsi, suivant l'invention, grâce au biseau ou chanfrein, l'insertion est moins traumatique vis-à-vis de la paroi du canal lacrymal et a un plus grand taux de réussite.

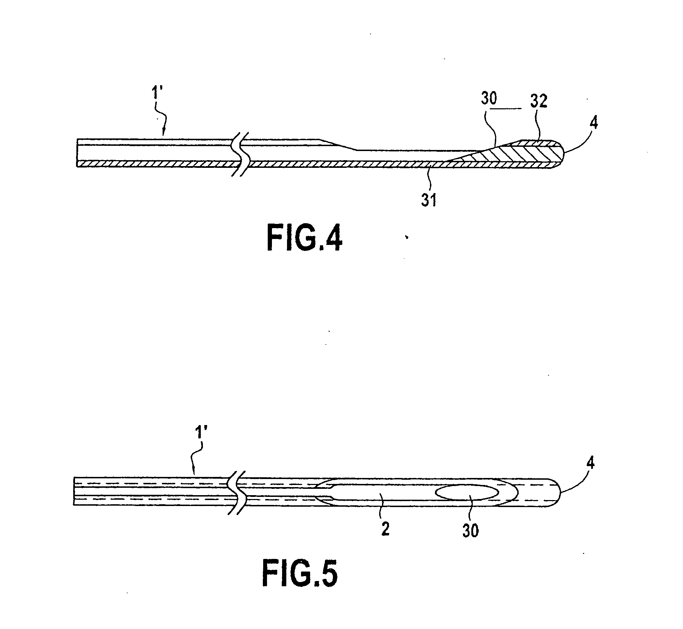

Aux figures 4 et 5, il est représenté une canule 1' suivant un autre mode de réalisation qui peut également être utilisée dans le système d'intubation décrit ci dessus à la place de la canule 1. Les parties identiques des deux canules 1 et 1' sont représentées par les mêmes références numériques.

Au delà de la lumière 2 et jusqu'à l'extrémité 4 distale opposée, la canule est pleine de sorte que le fil de guidage, lors de son insertion dans la canule, ne peut pas pénétrer dans la canule au delà de la lumière 2, venant buter contre la surface 30 intérieure de fond de la canule. Cette surface 30 intérieure est en biseau, en étant inclinée d'environ 18° par rapport à l'axe longitudinal de la canule.

La surface 30 intérieure formant le fond de la canule est plane. En coupe longitudinale, comme représenté à la figure 4, elle a la forme d'une droite qui s'étend d'un point 31 inférieur de la paroi intérieure latérale de la canule du côté opposé à la fente jusqu'à un point 32 supérieur de la paroi latérale de la canule du côté de la fente, le point 32 supérieur étant plus proche de l'extrémité 4 distale de la canule que le point 31. A la place d'une forme plane, on pourrait cependant prévoir une forme légèrement incurvée, concave ou convexe. La fonction de cette surface 30 formant le fond intérieur de la canule est de guider l'extrémité du fil de guidage pour le faire sortir par la lumière 2 lors de l'étape d'insertion du fil dans la canule, et l'empêcher ainsi de pénétrer dans une région de la canule au delà de la lumière 2, entre celle ci et l'extrémité distale, pour éviter, comme dans l'art antérieur, au fil d'y être coincé et obliger le chirurgien à aller rechercher ce fil bloqué dans l'espace au delà de la lumière.

Suivant un autre mode de réalisation non représenté, on peut prévoir non pas de remplir l'espace au delà de la lumière 2 mais de le fermer par une paroi qui constitue alors une surface de butée et de guidage pour le fil de guidage ayant la même fonction que la surface 30.

## Revendications

1. Canule (1 ; 11) destinée à être insérée dans les voies lacrymales pour y recevoir un fil solidarisé à une sonde que l'on souhaite installer dans le canal lacrymal, le fil étant guidé dans la canule pour ainsi traverser le canal lacrymal jusqu'à la sortie de ce dernier du côté de la fosse nasale, le chirurgien retirant alors la canule par l'autre sortie du canal (du côté des yeux) puis tirant le fil pour amener la sonde en position dans le canal lacrymal, la canule comportant un corps cylindrique creux oblong ouvert à une extrémité (3) et fermé à son autre extrémité (4) extérieure, une fente (5) latérale s'étendant à partir de l'extrémité ouverte vers l'extrémité extérieure fermée en se terminant par une lumière (2) latérale de plus grande dimension en largeur que la fente, la largeur étant mesurée dans la direction perpendiculaire à la direction longitudinale suivant laquelle s'étend la fente, la lumière (2) permettant le passage en son sein d'un fil de guidage introduit par l'ouverture de l'extrémité (3) ouverte dans le corps creux cylindrique pour un coulissement relatif par tirage de la canule et du fil de guidage, **caractérisée en ce que**
- la forme et la dimension de la canule sont telles que le fil de guidage, notamment son extrémité libre destinée à ressortir de la canule par la lumière (2), ne peut pas, une fois inséré dans la canule par l'ouverture de l'extrémité ouverte, pénétrer dans la partie de la canule au delà de la lumière (2) entre celle-ci et l'extrémité (4) extérieure fermée de la canule ;
- la canule comporte une paroi intérieure formant fond de la canule qui ferme l'espace entre la fin de la lumière dans la direction de l'extrémité extérieure fermée et cette extrémité extérieure fermée ; et
- la paroi intérieure de fond de la canule qui se trouve sensiblement au niveau de la fin de la lumière dans la direction longitudinale vers l'extrémité fermée extérieure fait un angle inférieur à 90°, notamment compris entre 15 et 30° avec l'axe longitudinal, pour ainsi former une rampe de guidage vers le haut, c'est à dire vers la lumière, pour l'extrémité du fil que l'on fait coulisser dans la canule en la poussant par l'ouverture de la canule.

2. Canule suivant la revendication 1, **caractérisée en ce que** l'espace entre la fin de la lumière dans la direction longitudinale vers l'extrémité extérieure fermée et l'extrémité extérieure fermée est plein de sorte que le fond intérieur de la canule se trouve sensiblement au niveau de la fin de la lumière.

3. Système dit de Ritleng comportant une canule suivant l'une des revendications 1 et 2 et une sonde d'intubation comportant au moins une partie en forme de tube (9) en un matériau souple biocompatible, notamment en silicone, destinée à être solidarisée à un fil (7) de guidage, notamment en prolène, dont une extrémité (16), pour solidariser mutuellement la sonde et le fil, est introduite dans le tube par une ouverture (22) de ce dernier, **caractérisée en ce que** le tube comporte un tronçon (24), du côté de l'ouverture (22) du tube par laquelle l'extrémité du fil est introduite dans le tube, ayant une forme en biseau, le diamètre extérieur du tube, le long du tronçon (24), augmentant depuis l'ouverture (22).

4. Système suivant la revendication 3, **caractérisé en ce que** l'épaisseur du tube augmente depuis l'ouverture du tube le long du tronçon en biseau puis est constante après cela.

## Patentansprüche

1. Kanüle (1; 11), die dazu bestimmt ist, in die Tränenwege eingesetzt zu werden, um einen Faden darin aufzunehmen, der mit einer Sonde, welche im Tränenkanal angebracht werden soll, fest verbunden ist, wobei der Faden in der Kanüle geführt ist, um so den Tränenkanal bis zu dessen Ausgang auf der Seite der Nasenhöhle zu durchqueren, wobei der Chirurg nun die Kanüle über den anderen Ausgang des Kanals (auf der Seite der Augen) herauszieht, dann den Faden zieht, um die Sonde im Tränenkanal in Position zu bringen, wobei die Kanüle einen länglichen zylindrischen Hohlkörper, der an einem Ende (3) offen und an seinem anderen äußeren Ende (4) geschlossen ist, umfasst, wobei ein seitlicher Schlitz (5) von dem offenen Ende zu dem geschlossenen äußeren Ende hin verläuft und dabei durch eine seitliche Öffnung (2) mit einer größeren Breitenabmessung als der Schlitz ausläuft, wobei die Breite in der Richtung senkrecht zu der Längsrichtung, entlang derer sich der Schlitz erstreckt, gemessen ist, wobei die Öffnung (2) den Durchgang, in ihrem Inneren, eines über die Öffnung des offenen Endes (3) in den zylindrischen Hohlkörper eingeführten Führungsfadens für ein Relativgleiten der Kanüle und des Führungsfadens durch Ziehen ermöglicht, **dadurch gekennzeichnet, dass**
- die Form und die Abmessung der Kanüle derart sind, dass der Führungsfaden, insbesondere sein freies Ende, das dazu bestimmt ist, aus der Kanüle über die Öffnung (2) auszutreten, nicht in den Teil der Kanüle jenseits der Öffnung (2), zwischen dieser und dem geschlossenen äußeren Ende (4) der Kanüle eindringen kann, sobald er über die Öffnung des offenen Endes in die Kanüle eingefügt ist;
- die Kanüle eine einen Boden der Kanüle bildende Innenwand umfasst, die den Raum zwischen dem Ende der Öffnung in der Richtung des geschlossenen äußeren Endes und diesem geschlossenen äußeren Ende verschließt; und
- die innere Bodenwand der Kanüle, die sich im Wesentlichen im Bereich des Endes der Öffnung in der Längsrichtung zu dem geschlossenen äußeren Ende hin befindet, einen Winkel kleiner als 90°, insbesondere zwischen 15 und 30° mit der Längsachse bildet, um so eine Aufwärtsführungsrampe, das heißt zu der Öffnung hin, für das Ende des Fadens zu bilden, das man - unter dessen Schieben aus der Öffnung der Kanüle - in der Kanüle gleiten lässt.

2. Kanüle nach Anspruch 1, **dadurch gekennzeichnet, dass** der Raum zwischen dem Ende der Öffnung in der Längsrichtung zu dem geschlossenen äußeren Ende hin und dem geschlossenen äußeren Ende massiv ist, so dass der Innenboden der Kanüle sich im Wesentlichen im Bereich des Endes der Öffnung befindet.

3. Sogenanntes Ritleng-System, umfassend eine Kanüle nach einem der Ansprüche 1 und 2 und eine Intubationssonde mit wenigstens einem röhrenförmigen Teil (9) aus einem biokompatiblen flexiblen Material, insbesondere aus Silikon, der dazu bestimmt ist, mit einem Führungsfaden (7), insbesondere aus Prolene, fest verbunden zu werden, dessen eines Ende (16) zum gegenseitigen festen Verbinden der Sonde und des Fadens in die Röhre über eine Öffnung (22) derer eingeführt ist, **dadurch gekennzeichnet, dass** die Röhre einen Abschnitt (24) auf der Seite der Öffnung (22) der Röhre, über die das Ende des Fadens in die Röhre eingeführt wird, mit einer abgeschrägten Form umfasst, wobei der Außendurchmesser der Röhre entlang des Abschnitts (24) von der Öffnung (22) aus zunimmt.

4. System nach Anspruch 3, **dadurch gekennzeichnet, dass** die Dicke der Röhre von der Öffnung der Röhre aus entlang des abgeschrägten Abschnitts zunimmt, anschließend hiernach konstant ist.

## Claims

1. Cannula (1 ; 11) designed to be inserted into tear ducts for receiving a wire connected to a probe that is to be inserted into the tear duct, the wire being guided into the cannula so as to traverse the tear duct in this way up to the exit of the latter on the side of the nasal cavity, the surgeon then removing the cannula through the other exit of the duct (on the side of the eyes) then pulling the wire to bring the probe into position in the tear duct, the cannula comprising an oblong, hollow cylindrical body open at one end (3) and closed at its other outer end (4), a lateral slot (5) extending from the open end towards the closed outer end ending with a lateral opening (2) with a larger width dimension than the slot, the width being measured in a direction perpendicular to the longitudinal direction along which the slot extends, the opening (2) allowing the passage within it of a guide wire introduced through the opening of the open end (3) into the cylindrical hollow body for a relative sliding by pulling of the cannula and the guide wire, **characterised in that**
- the form and the dimension of the cannula are such that the guide wire, in particular its free end designed to come out of the cannula through the opening (2), cannot, once inserted into the cannula through the opening of the open end, penetrate into the part of the cannula beyond the opening (2) between the latter and the closed outer end (4) of the cannula ;
- the cannula comprises an internal wall forming the base of the cannula which closes the spacing between the end of the opening in the direction of the closed outer end and said closed outer end ; and
- the internal wall of the base of the cannula which is located substantially at the level of the end of the opening in the longitudinal direction towards the outer closed end makes an angle lower than 90°, in particular comprised between 15 and 30°, with the longitudinal axis, so as to form a guiding ramp toward the top, namely towards the opening, for the wire end which is made to slide in the cannula by pushing it through the opening of the cannula.

2. Cannula according to claim 1, **characterised in that** the spacing between the end of the opening in the longitudinal direction towards the closed outer end and the closed outer end is solid such that the inner base of the cannula is located substantially at the end of the opening.

3. Ritleng type system comprising a cannula according to claims 1 and 2, and an intubation probe comprising at least one part in the form of a tube (9) made of a biocompatible flexible material, in particular made of silicone, designed to be connected to a guide wire (7), in particular made of prolene, one end (16) of which for joining together the probe and the wire, is introduced into the tube through an opening (22) of the latter, **characterised in that** the tube comprises a section (24), from the side of the opening (22) of the tube through which the end of the wire is introduced into the tube, having a bevelled form, the exterior diameter of the tube, along the section (24), increasing from the opening (22).

4. System according to claim 3, **characterised in that** the thickness of the tube increases from the opening of the tube along the bevelled section and then is constant thereafter.
